# EUROPEAN PATENT SPECIFICATION

(11) **EP 3 655 755 B1**
(45) Date of publication and mention of the grant of the patent: **30.08.2023**
(21) Application number: 18835598.6
(22) Date of filing: 18.07.2018
(51) Int. Cl.: G01N 19/10, G01N 33/24, A01G 27/00, F16K 31/00

(54) **SOIL WATER POTENTIAL EFFECTOR APPARATUS**
BODENWASSERPOTENTIALEFFEKTORVORRICHTUNG
APPAREIL DE MISE EN OEUVRE DE POTENTIEL HYDRIQUE DANS LE SOL

(30) Priority: 18.07.2017 IL 25354017; 26.04.2018 US 201862662950 P
(43) Date of publication of application: 27.05.2020
(73) Proprietor: I-Dripper Ltd., 7319600 Kfar Ruth (IL)
(72) Inventor: SHETRIT, Arik, 7313400 Kfar Haornim (IL)
(74) Representative: Lecomte & Partners
(86) International application number: PCT/IL2018/050792
(87) International publication number: WO 2019/016807

(56) References cited:
- WO-A1-98/04915
- WO-A1-2018/067625
- DE-A1-102011 056 754
- JP-A- S63 200 062
- JP-A- 2014 041 054
- SU-A1- 966 571
- US-A- 3 871 211
- US-A- 4 130 012
- US-A- 4 655 076
- US-A- 5 329 081
- US-A1- 2008 041 170
- US-A1- 2008 202 219
- US-B1- 6 782 909
- US-B1- 6 782 909
- US-B1- 7 181 953
- Anonymous: "ALCOSORB SWELL GEL 1Kg", , 1 January 2021 (2021-01-01), pages 1-5, XP055783186, Retrieved from the Internet: URL:https://www.dejex.co.uk/shop/horticult ural-substrates-compost-and-bark-additives -16/perlite-vermiculite-and-wetting-agents -for-horticulture-18/alcosorb-400-swell-ge l-1kg [retrieved on 2021-03-08]

## Description

### TECHNICAL FIELD OF THE INVENTION

The invention relates generally to the field of soil water potential, and in particular to soil water potential effector systems and their applications in agriculture.

### BACKGROUND OF THE INVENTION

Water content in the soil can be directly determined using the difference in weight before and after drying a soil sample. This direct technique is usually referred to as the thermogravimetric method (or simply gravimetric) when expressing water content as weight of water overweight of dry soil, GWC[lb³ lb^{~3}] (i.e., the ratio of the mass of water present in a sample to the mass of the soil sample after it has been oven-dried (100-110°C) to a constant weight). On the other hand, the thermo-volumetric method (or simply volumetric) gives the water content as the volume of water in a volume of undisturbed soil VWC[lb³ lb^{~3}] (i.e., volume of water related to the volume of an oven-dried undisturbed sample (soil core)). Although these direct methods are accurate (+0.01 lb³ lb^{~3}) and inexpensive, they are destructive, slow (2 days minimum), time-consuming and do not allow for making repetitions in the same location.

Alternatively, many indirect methods are available for monitoring soil water content. These methods estimate soil moisture by a calibrated relationship with some other measurable variable. The suitability of each method depends on several issues such as cost, accuracy, response time, installation, management and durability.

Depending on the quantity measured, indirect techniques are first classified into volumetric and tensiometric methods. While the former gives volumetric soil moisture, the latter yields soil suction or water potential (i.e., tension exerted by capillarity). Volumetric technique estimates the volume of water in a sample volume of undisturbed soil [lb³ lb^{~3}]. This quantity is useful for determining how saturated the soil is (i.e., fraction of total soil volume filled with the soil aqueous solution). When it is expressed in terms of depth (i.e., volume of water in soil down to a given depth over a unit surface area (inches of water)), it can be compared with other hydrological variables like precipitation, evaporation, transpiration, and deep drainage.

Tensometric methods estimate the soil water matric potential that includes both adsorption and capillary effects of the soil. The matric potential is one of the components of the total soil water potential that also includes gravitational (position with respect to a reference elevation plane), osmotic (salts in soil solution), gas pressure or pneumatic (from entrapped air), and overburden components. The sum of matric and gravitational potentials is the main driving force for water movement in soils and other soil-like porous media.

All available tensiometric instruments have a porous material in contact with the soil, through which water can move. Thereby, water is drawn out of the porous medium in a dry soil and from the soil into the medium in a wet soil. Along with certain advantages such as direct reading and salinity resistance, tensiometers are known to have limited soil suction range (<1 bar); relatively slow response time; they require intimate contact with soil around the ceramic cup for consistent readings and to avoid frequent discharge (breaking of water column inside), requires frequent maintenance (refilling) to keep the tube full of water, especially in hot dry weather.

US 5,329,081 discloses an apparatus comprising a housing with at least one portion that is water permeable, a volume changing material VCM made of gel beads, capable of retaining water, wherein said VCM increases of decreases its volume in response to increased or decreased water concentration respectively, and wherein said VCM is in direct contact with an inner surface of said at least one water permeable portion of said housing, a membrane contacting the VCM, and a piston configured to engage the VCM via the membrane above a predetermined threshold of water concentration and configured to transduce a signal when engaged or disengaged.
SU 966 571 A1, JP 2014-041054 A, US 6,782,909 B1, WO 2018/067625 A1 and WO 98/04915 A1 disclose various soil water content measuring apparatuses.

Thus, there remains a need for a water potential effector system which is universal, long-lasting; durable, inexpensive and responsive to subtle changes in soil water content.

### SUMMARY OF THE INVENTION

Accordingly, it is a principal object of the present invention to overcome disadvantages of the prior art methods and systems for sensing and measuring soil water potential providing a universal, durable, cost effective, broad range water potential effector apparatus having fast response time and capable of responding to subtle changes in soil water content.

The water potential effector apparatus of the invention is according to claim 1.

Further developments of the invention are according to dependent claims 2 to 7.

According to some embodiments, the receiver comprises a pressure sensor, a traveling sensor, a force sensor, a valve, or a gauge.

According to some embodiments, the porous media is a natural, synthetic, or a semisynthetic membrane.

According to some embodiments, the compressible insert comprises a gas-filled inner volume.

According to some embodiments, the VCM comprises a biocide. According to some embodiments, the hydrogel is a biocide.

According to some embodiments, the apparatus of the invention further comprises a diaphragm between the VCM, insert or both and the receiver, wherein the diaphragm transfers force from the VCM, insert or both to the receiver.

According to some embodiments, the apparatus is operatively linked to an irrigation system such that the irrigation system activates or shuts off in response to the transduced signal. According to some embodiments, a device is disclosed comprising a soil potential measuring system and the apparatus of the invention.

According to some embodiments, the apparatus of the invention is for use in effecting the water potential in water-containing media proximal to the apparatus.

Additional features and advantages of the invention will become apparent from the following drawings and description.

### BRIEF DESCRIPTION OF THE DRAWINGS

For a better understanding of the invention and to show how the same may be carried into effect, reference will now be made, purely by way of example, to the accompanying drawings in which like numerals designate corresponding elements or sections throughout.

With specific reference now to the drawings in detail, it is stressed that the particulars shown are by way of example and for purposes of illustrative discussion of the preferred embodiments of the present invention only and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice. In the accompanying drawings:
Figures 1A-1E show a moisture sensor apparatus comprising a liquid permeable container and a transducer, according to an example not forming part of the present invention (Figure 1A) and embodiments (Figures 1B-1E) of the disclosed subject matter.
Figures 2A-2C show a moisture sensor comprising a wireless transmitter and an antenna, according to exemplary embodiments of the disclosed subject matter.
Figures 3A-3D show a moisture sensor embedded in an irrigation apparatus, according to exemplary embodiments of the disclosed subject matter.
Figures 4A-4C show a moisture sensor comprising a pressure sensor, according to exemplary embodiments of the disclosed subject matter.
Figure 5 shows multiple moisture sensors located in multiple depths in a specific area, according to exemplary embodiments of the disclosed subject matter.
Figure 6 shows multiple moisture sensors located in multiple locations in a specific area, according to exemplary embodiments of the disclosed subject matter.
Figures 7A-B show a size-adjustable moisture sensor, according to exemplary embodiments of the disclosed subject matter.

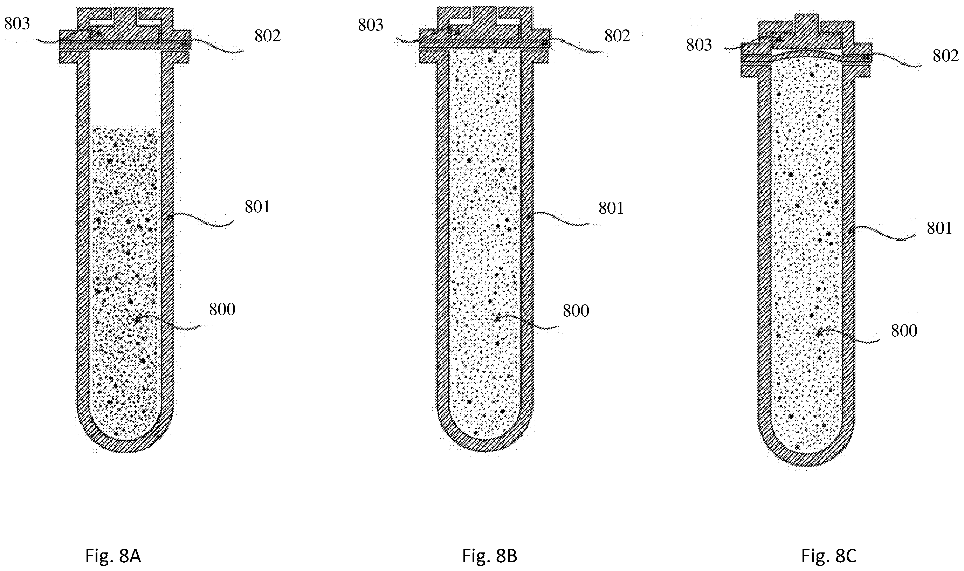

Figures 8A-C: Diagrams illustrating a cross-section of the water potential effector apparatus not forming part of the present invention without compressible insert. (8A) Diagram illustrating the apparatus when VCM [800] is not fully swollen. (8B) Diagram illustrating the apparatus when the VCM [800] is swollen. (8C) Diagram illustrating the apparatus when the VCM is fully swollen and the force is applied to the diaphragm [802] and the receiver [803].
Figures 9A-B: Diagrams illustrating a cross section of the water potential effector apparatus with a compressible insert [905]. The opening of the compressible insert is oriented toward the bottom part of the housing [901]. (9A) Diagram illustrating the apparatus when VCM [900] is swollen and the compressible insert is not compressed. (9B) Diagram showing the VCM fully swollen and the insert compressed.
Figures 10A-B: Diagrams illustrating a cross section of the water potential effector apparatus with a compressible insert [1005]. The opening of the compressible insert is oriented toward the top of the housing [1001] and operationally directly engaged with the receiver [1003]. (10A) Diagram illustrating the apparatus when VCM [1000] is swollen and the compressible insert is not compressed. (10B) Diagram showing the VCM fully swollen and the insert compressed.
Figures 11A-B: Diagrams Illustrating a cross section of the water potential effector apparatus with a compressible insert [1105]. The opening of the compressible insert is oriented toward the top of the housing [1101] and operationally engaged with the receiver [1103] through a diaphragm [1102]. (11A) Diagram illustrating the apparatus when VCM [1100] is swollen and the compressible insert is not compressed. (11B) Diagram showing the VCM fully swollen and the insert compressed.

### DETAILED DESCRIPTION OF THE INVENTION

Before explaining at least one embodiment of the invention in detail, it is to be understood that the invention is not limited in its application to the details of construction and the arrangement of the components set forth in the following description or illustrated in the drawings. The invention is applicable to other embodiments or of being practiced or carried out in various ways. Also, it is to be understood that the phraseology and terminology employed herein is for the purpose of description and should not be regarded as limiting.

The present invention discloses a moisture sensor apparatus comprising a container made of a liquid permeable material designed to be inserted into a surface, such as the ground. The container contains a volume changing material (VCM) configured to change its volume in response to absorbing liquid permeating via the container. The liquid may be water. Optionally, the apparatus also comprises a second unit removably coupled to the container. The second unit comprises a transducer configured to generate an electrical signal representing a force being measured by the transducer. The transducer may be a load cell, a pressure sensor and the like. The apparatus may also comprise a piston operatively coupled to the VCM, the piston moves towards the transducer and applies a force on the transducer when the VCM swells. The transducer is configured to measure the force or pressure applied by the piston and to generate an electrical signal representing the measured force. In some exemplary embodiments, the apparatus may comprise a wireless transmitter for transmitting the electrical signal to a remote destination. The optional elements described herein are not limited merely to the figures or embodiments in which they are listed, but rather may be applied or combined with any figures or embodiments herein described.
also It is also disclosed a method for receiving electrical signals from multiple moisture sensor apparatuses and analyze them. Such analysis may comprise generating a moisture map according to locations of the moisture sensors. The second unit may comprise a transducer cover secured to the container, for example via a screw, bolt, hook and loop, Velcro and the like. The second unit can be easily separated from the container, enabling a person installing and using the apparatus to adjust the depth of the container in the surface. Similarly, adjusting the depth of the container may also be achieved by adding an intermediate hollow element between the transducer and the wireless transmitter, which may be required to be placed outside the surface, for example above the ground. The modularity achieved by the second unit being removable from the container enables installing different containers, according to specific needs, for example changing a container according to the season, type of liquid absorbing volume (LAV) it is inserted in, type of transducer, and the like.

In some exemplary cases, the moisture sensor of the present invention is embedded in an apparatus for controlling the water supplied to the vicinity of the apparatus according to swelling of the VCM. The apparatus can thus be used for irrigation. The irrigation apparatus is coupled with a water source and inserted into the surface in a location which needs to be irrigated, to control the level of water content in the surface at said location. The surface may be ground, for example grass or soil or a surface on which plants or mushrooms grow. In some other cases, the location in the surface can comprise bacteria, microorganisms, or mushrooms which need to be irrigated. In some cases, the location in the ground may comprise animals, for example snails in a snail farm which require a moist soil.

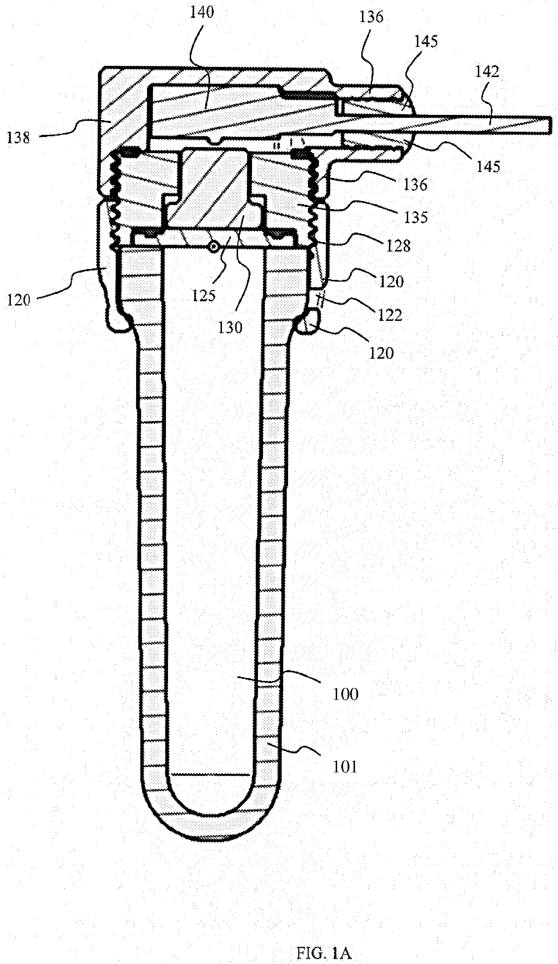

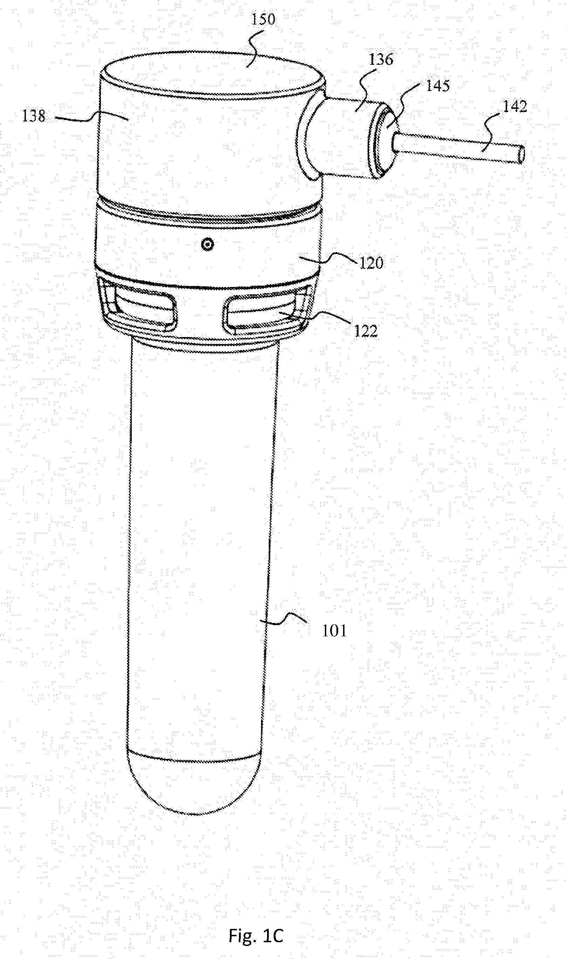

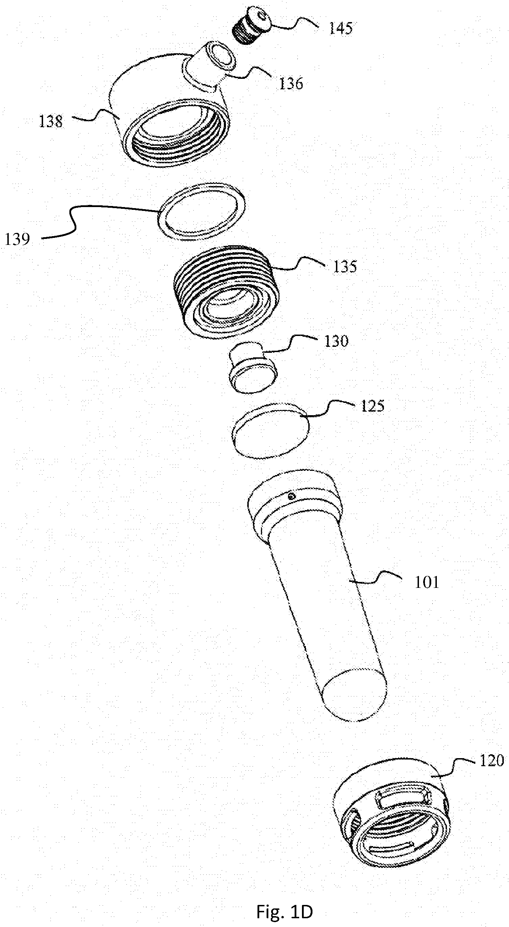

Figures 1A-1E show a moisture sensor apparatus comprising a liquid permeable container and a transducer, according to exemplary embodiments of the disclosed subject matter. Figure 1A shows a cross- sectional view, figure 1C shows an assembled view of the moisture sensor, figure 1D shows an exploded view of the apparatus and figure IE shows an exploded view of the transducer and the transducer housing. Figure IB shows the same cross-sectional view as 1A, but with the addition of a compressible insert 105. Reference is now made to figures 1A-E.

The liquid container 101 may be inserted into a surface, or LAV, such as a ground, in order to enable the moisture sensor to sense the moisture level of the surface. Liquid container 101 is a housing for the moisture sensor apparatus. Liquid, such as water, permeates from the surface and comes in contact with the VCM 100 located in the liquid container 101. The liquid container 101 may be made of any water permeable material, such as for a non-limiting example, clay. Only a portion of the container 101 may be water permeable. The VCM 100 swells when contacting the liquid and pushes piston 130 away from the liquid container 101, and towards a transducer 140. The transducer senses the force applied by the piston 130 and converts the force into an electrical signal. The electrical signal may be transmitted from the transducer 140 via a cable located in tube 142 to a transmitter that sends the signal to a remote location. In some other cases, the electrical signal may be saved on a memory unit communicating with the transducer 140. In some embodiments, the transducer is a receiver that receives the signal from the VCM 100 or insert 105.

In some cases, the moisture sensor comprises a membrane 125 located between the VCM 100 and the piston 130, for preventing leakage of the VCM 100 into the electrical components located above the piston 130, for example the transducer 140. The membrane 125 may be attached to the piston 130 and move when the piston 130 moves. The piston 130 may move inside a piston housing 135. The inner sidewalls of the piston housing 135 guide the piston 130 towards the transducer 140 and back towards the VCM 100. In some exemplary cases, the external sidewalls of the piston housing 135 comprise a screw thread 128. In some embodiments, the membrane is part of a diaphragm. In some embodiments, the membrane is a diaphragm.

In some exemplary embodiments, the apparatus comprises a container cover 120 covering an upper portion of the liquid container 101, towards the piston housing 135. The container cover 120 may be made of a rigid or semi-rigid material such as plastics or metal. In some exemplary embodiments, the upper portion of the liquid container 101 is wider than a narrower, lower portion of the liquid container 101. In such embodiments, the container cover 120 is slid from the bottom of the liquid container 101 upwards. Optionally, the container cover 120 may have one or more niches 122 via which liquid may permeate the liquid container 101. The container cover 120 may assist in securing the liquid container 101 to the transducer 140. That is, by threading the upper inner sidewalls of the container cover 120 into the lower portion of the screw thread 128. Similarly, the upper portion of the screw thread 128 is configured to be threaded to a transducer housing 138. The transducer housing 138 houses the transducer 140 and may be attached to the piston housing 135 via screw thread 128. In some cases, the transducer housing 138 may be attached to the piston housing 135 using adhesive materials, bolts and the like.

The transducer housing 138 may also be attached to a gasket 145. The gasket 145 may be used in case the cable passing inside tube 142 is configured to reach a component located outside the moisture sensor. In such a case, the gasket is configured to seal the aperture of the transducer housing 138 via which the cable exits towards a remote device. Such a remote device may be another sensor, a server, a transmitter and the like. A lateral section 136 of the transducer housing 138 is configured to house the gasket 145.

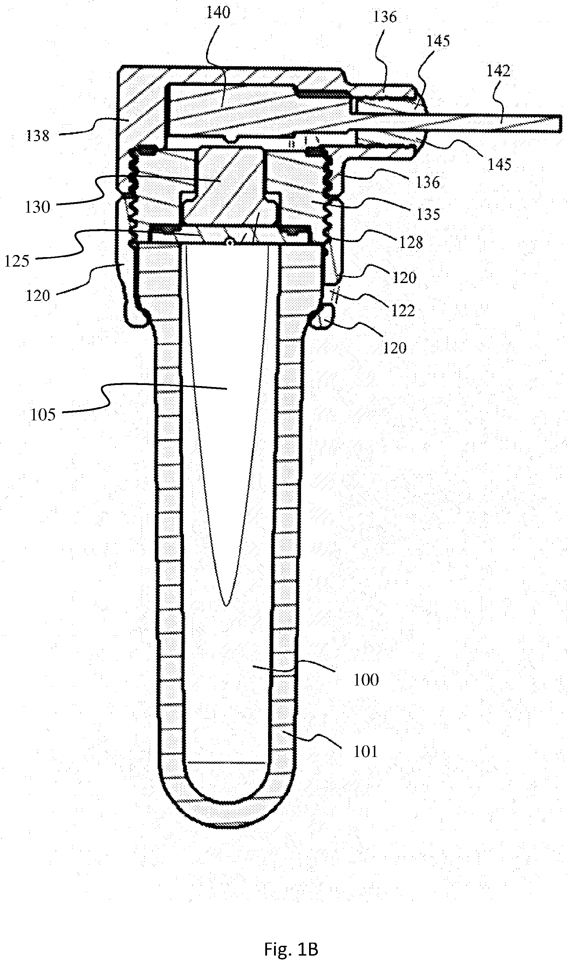

Figure 1B is identical to figure 1A, but also shows a compressible insert 105 that maintains at least a predetermined inner pressure with the apparatus. In some exemplary embodiments insert 105 directly contacts membrane 125. In some embodiments, insert 105 directly contacts piston 130. In some embodiments, insert 105 directly contact transducer 140. In some embodiments, insert 125 is anchored to container 101. In some embodiments, insert 105 is free floating in VCM 100.

Figure 1C also shows an upper seal 150 for preventing materials and liquids to penetrate the moisture sensor and touch the transducer 140. In some exemplary cases, the upper seal 150 is an integral part of the transducer housing 138. In some exemplary cases, the lateral section 136 is an integral part of the transducer housing 138. Figure ID introduces a ring 139 connecting the transducer housing 138 and the piston housing 135.

Figure IE shows an exploded view of the moisture sensor. The exploded view shows a screw 145 that secures the tube 142 to the transducer 140. The screw 145 has a hollow void in which the tube 140 is positioned when attached to the transducer. The screw 145 may define the direction of the tube 142, and thus the direction of the cable that transfers the electrical signal. The cable and the tube 142 are secured to the transducer 140 via a hollow void inside the lateral section 136 of the transducer housing 138.

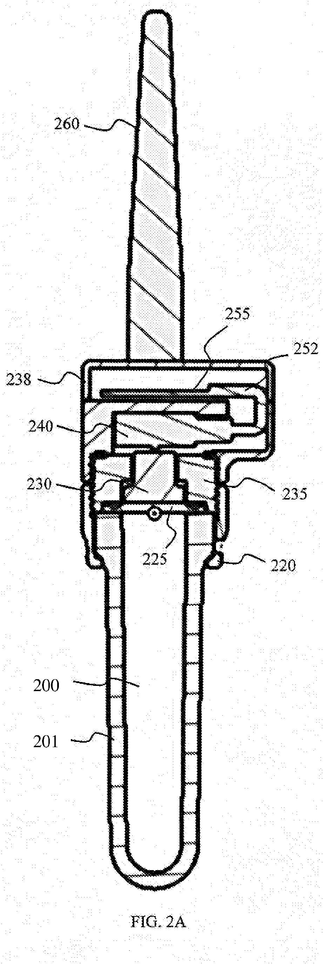

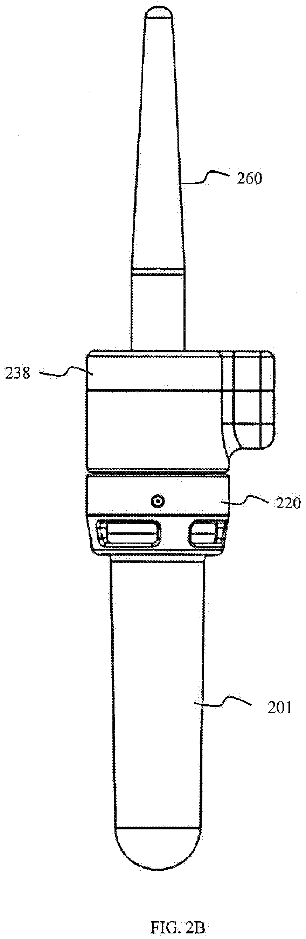

Figures 2A-2C show a moisture sensor comprising a wireless transmitter and an antenna, according to exemplary embodiments of the disclosed subject matter. The moisture sensor comprises a liquid container 201 that contains VCM 200. The liquid container 201 may be covered, at least partially, by container cover 220. Piston 230 moves towards transducer 240 according to change in the volume of the VCM 200, according to liquid permeating the liquid container 201. A membrane 225 may separate the VCM 200 from the piston 230. The piston 230 moves in a piston housing 235, surrounding the volume in which the piston 230 moves. The piston 230 is in contact with the transducer 240 that measures the force applied by the piston 230 and generates an electrical signal representing the measured force. The transducer 240 is located in a transducer housing 238. The transducer housing 238 may be attached to the piston housing 235, for example via adhesives, bolts and the like. In some cases, the external sidewalls of the piston housing 235 comprise a screw thread, to which both the container cover 220 and the transducer housing 238 are screwed.

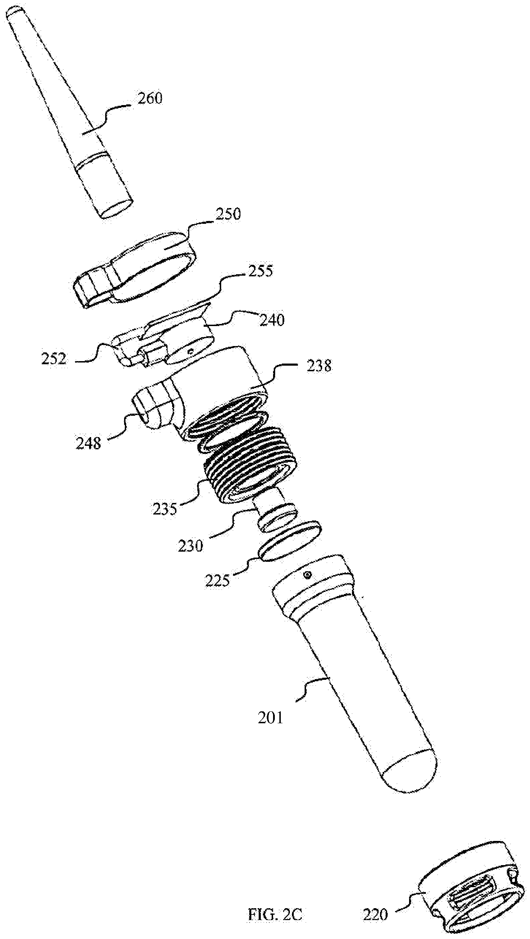

The moisture sensor of figures 2A-2C discloses a wireless transmitter 255 connected to the transducer 240. In such a case, the wireless transmitter 255 may be located inside the transducer housing 238. The wireless transmitter 255 is configured to transmit the electrical signal to a remote location, for example to a cloud storage, an internet gateway, a server communicating with multiple moisture sensors, and other sensors at the same network and the like. The manner of transmitting the electrical signal, for example the specific network, communication protocol, frequency band and the like may be selected by a person skilled in the art. The wireless transmitter 255 may be physically attached to the transducer 240 and receive the electrical signal via connectors. In some other cases, the wireless transmitter 255 may be connected to the transducer 240 via communication cable 252. The communication cable 252 may be made of coaxial cable. In some exemplary cases, the wireless transmitter 255 may be secured to the moisture sensor and located externally from the transducer housing 238. In some cases, the wireless transmitter 255 may be removable or replaceable from the moisture sensor. In some cases, the wireless transmitter 255 may be connected to an antenna 260 that outputs the electrical signal. The wireless transmitter 255 may process the electrical signal received from the transducer 240, for example add a destination address to the electrical signal or reformat the electrical signal according to a format that fits the antenna 260 or the signal destination or network. In some cases, the transducer housing 238 has a lateral extension 248 as shown in figure 2C. The lateral extension is configured to house the communication cable 252 in case the communication cable 252 extends laterally, further than the circular form of the transducer housing 238. In some cases, the lateral extension 248 may be removable from the transducer housing 238 according to the size and architecture of the communication cable 252, the transducer 240 and the wireless transmitter 255. In case the lateral extension 248 is removed, a gasket may be used to seal the hole caused by the removal. Embodiments depicted in figures 2A-C are performed with a compressible insert, such as has been described herein throughout.

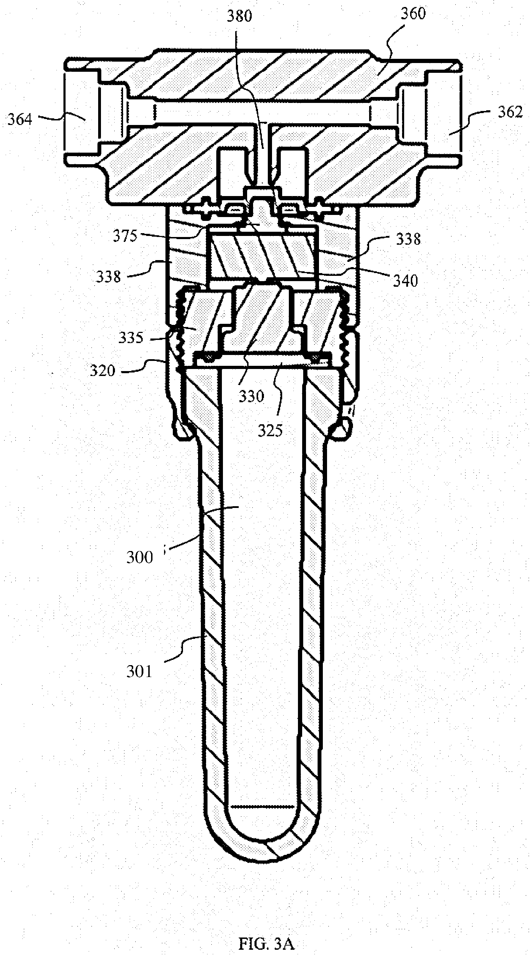

Figures 3A-3D show a moisture sensor embedded in an irrigation apparatus, according to exemplary embodiments of the disclosed subject matter. The moisture sensor comprises a liquid container 301 that contains VCM 300. The liquid container 301 may be covered, at least partially, by container cover 320. Piston 330 moves towards transducer 340 according to change in the volume of the VCM 300, according to liquid permeating the liquid container 301. A membrane 325 may separate the VCM 300 from the piston 330. The piston 330 moves in a piston housing 335, surrounding the volume in which the piston 330 moves. The piston 330 is in contact with the transducer 340 that measures the force applied by the piston 330 and generates an electrical signal representing the measured force. The transducer 340 is located in a transducer housing 338. The transducer housing 338 may be attached to the piston housing 335, for example via adhesives, bolts and the like. In some cases, the external sidewalls of the piston housing 335 comprise a screw thread, to which both the container cover 320 and the transducer housing 338 are screwed.

The irrigation apparatus comprises a water inlet 364 connected to a water pipe. The water pipe may provide water to multiple irrigation apparatuses. The transducer 340 is operatively coupled to a secondary piston 375. When the piston 330 moves towards the transducer 340, the transducer also moves, towards the secondary piston 375. The transducer's movement may be in the range of 0.1-20 millimeters. The transducer 340 causes the secondary piston 375 to move upwards, thus blocking water from moving towards an irrigation output 385 of the irrigation apparatus. Water may flow from the water inlet 364 towards irrigation output via secondary passageway. Thus, when the secondary piston 375 to move upwards, according to the volume of the VCM 300 in the container 301, water cannot flow via the secondary passageway to the irrigation output 385. When the volume of the VCM 300 decreases, the piston 330 moves downwards, and as a result the transducer 340 and the secondary piston 375 move downwards, allowing water to flow via the secondary passageway to the irrigation output 385.

The irrigation apparatus comprises an irrigation housing 360 configured to house the water inlet 364, irrigation outlet 385 and the secondary passageway. In some exemplary cases, the irrigation housing 360 further comprises a connector output 362 configured to transfer water from the water inlet 364 to another apparatus, for example another water pipe or another irrigation apparatus. The irrigation housing 360 may be attached to the transducer housing 338, for example via bolt 390 inserted in a niche in the bottom portion of the irrigation housing 360. In some other exemplary cases, the irrigation housing 360 may be attached to the piston housing 335 or to the container cover 320.

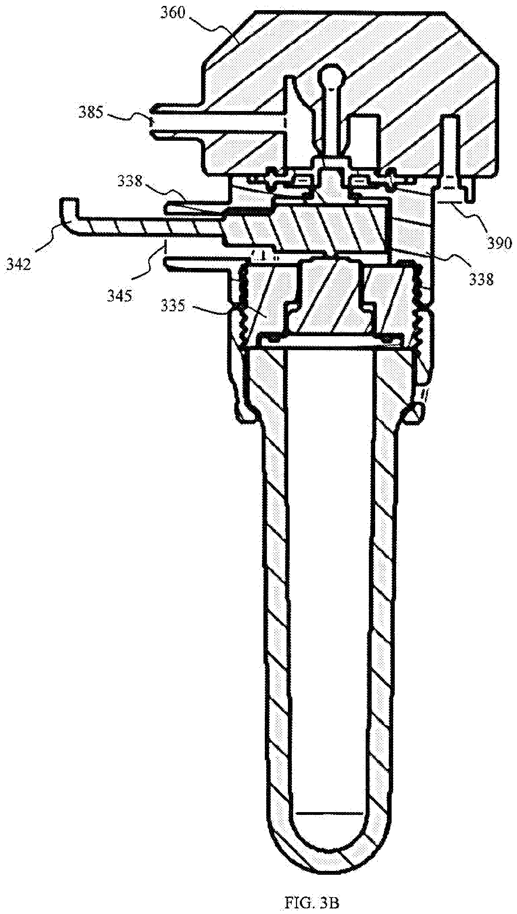

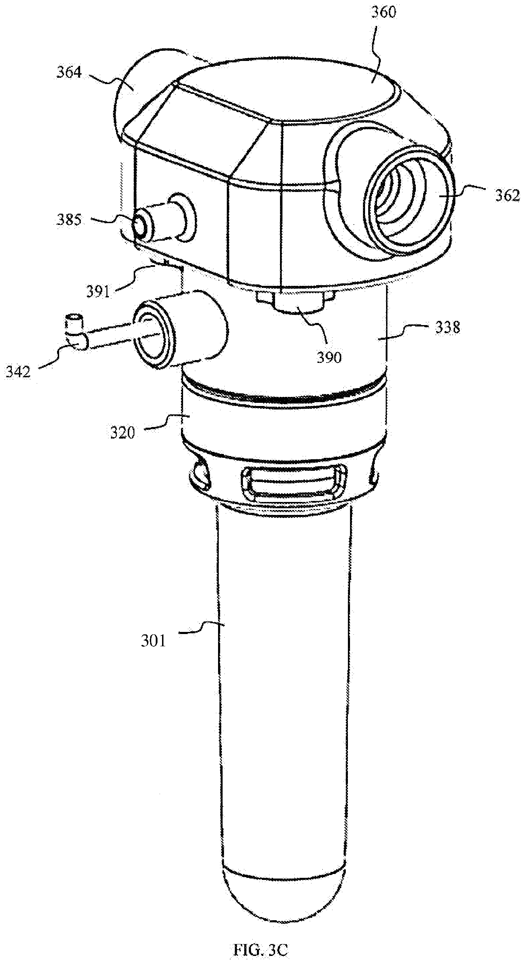

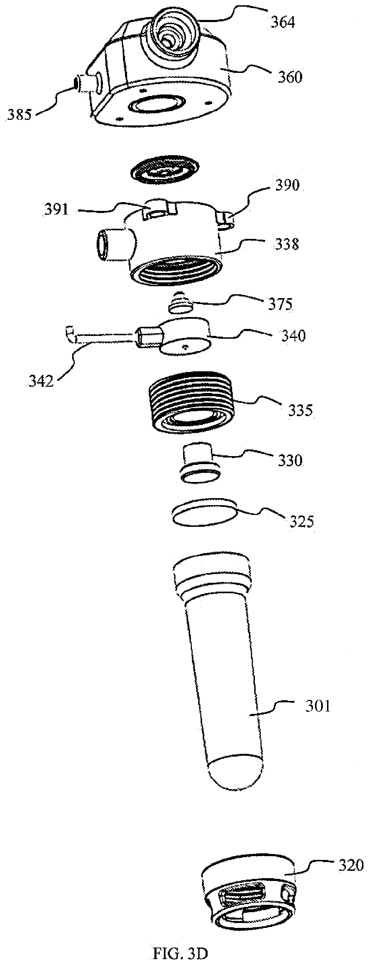

Figure 3B shows a lateral cross section of the irrigation apparatus with the moisture sensor. The lateral cross section shows the communication tube 342 connected to the transducer 340 and carrying the electrical signal out of the irrigation apparatus. The hole formed to allow the communication tube 342 to extend outwards of the transducer housing 338 may be sealed by gasket 345. Figure 3C shows the irrigation apparatus assembled. The exploded view of figure 3D shows two holes 390, 391 located at the upper portion of the transducer housing 338, securing the irrigation housing 360 to the transducer housing 338. Embodiments depicted in figures 3A-D may also be performed with a compressible insert, such as has been described herein throughout.

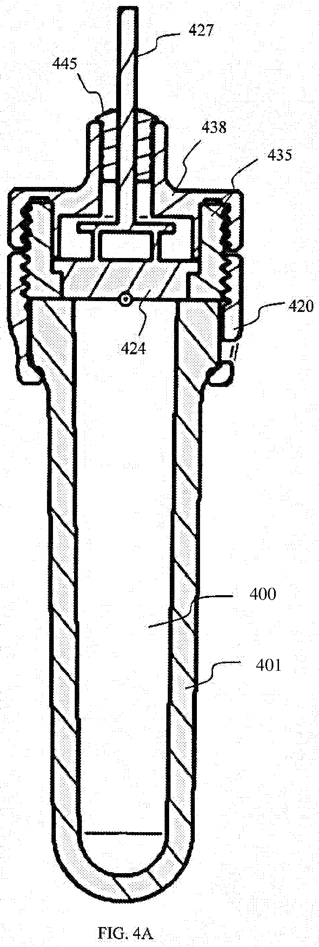

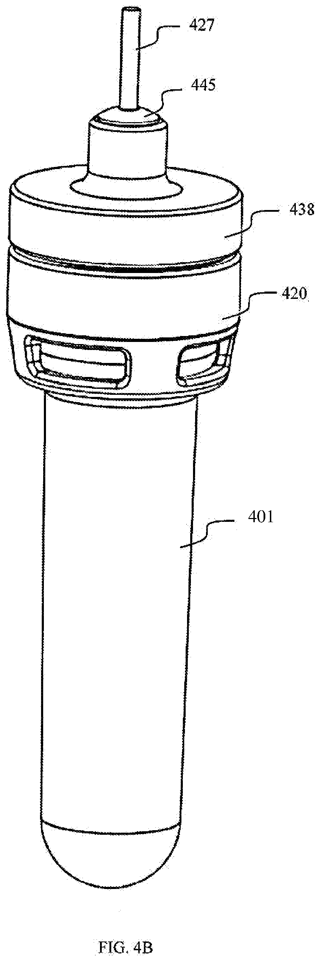

Figures 4A-4C show a moisture sensor comprising a pressure sensor, according to exemplary embodiments of the disclosed subject matter. The moisture sensor comprises a liquid container 401 that contains VCM 400. The liquid container 401 may be covered, at least partially, by container cover 420. Pressure transducer 424 detects changes in the volume of the VCM 400, according to liquid permeating the liquid container 401. The pressure transducer 424 moves in a piston housing 435, surrounding the volume in which the pressure transducer 424 moves. The pressure transducer 424 measures the pressure applied directly by the VCM 400 and generates an electrical signal representing the measured pressure. A communication tube 427 is connected to the pressure transducer 424 and enables to carry the electrical signal out of the moisture sensor. The communication tube 427 may be located in a transducer housing 438. The transducer housing 438 may be attached to the piston housing 435, for example via adhesives, bolts and the like. In some cases, the external sidewalls of the piston housing 435 comprise a screw thread, to which both the container cover 420 and the transducer housing 438 are screwed. The hole in the transducer housing 438 caused by the communication tube 427 may be sealed by gasket 445. The hole may be located in the ceiling of the transducer housing 438 or in sidewalls of the transducer housing 438.

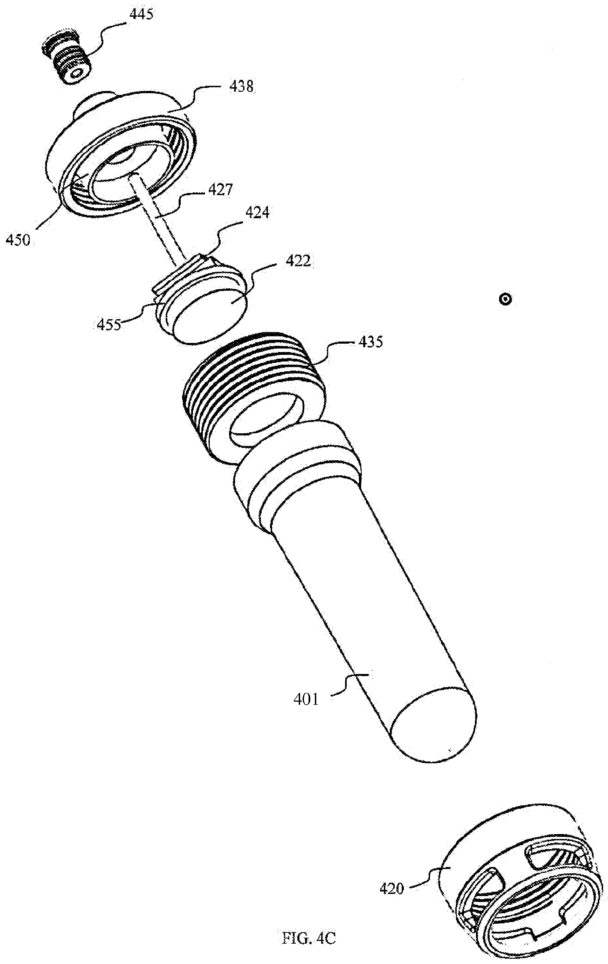

Figure 4C shows an exploded view of the moisture sensor having the pressure transducer 424. The exploded view shows a transducer base 422 configured to be inserted into a hole in the piston housing 435. The transducer base 422 is attached to a wider block 455. The block 455 is wider than the hole in the piston housing 435 and limits the downward movement of the pressure transducer 424, towards the liquid container 401. In some exemplary cases, the transducer housing 438 comprises an inner ring 450 configured to secure the block 455. Embodiments depicted in figures 4A-C are also performed with a compressible insert, such as has been described herein throughout.

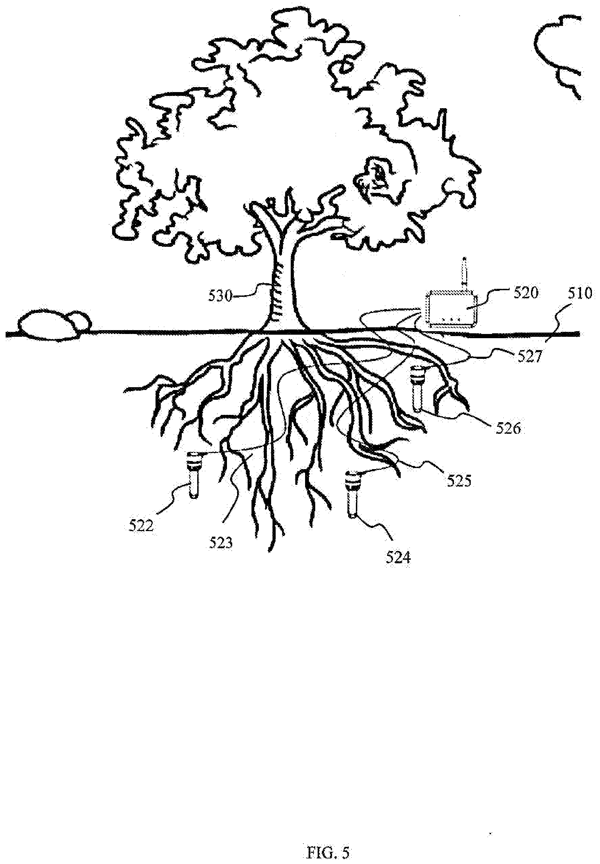

Figure 5 shows multiple moisture sensors located in multiple depths in a specific area, according to exemplary embodiments of the disclosed subject matter. Moisture sensors 522, 524, 526 transfer the electrical signals, which represent the force applied by the VCM, to a remote device 520. The remote device 520 may be able to store the signals, process the signals, generate periodic reports, generate maps that indicate moisture levels in various locations in a certain area and the like. The remote device 520 may comprise a wireless transmitter for transmitting the signals or data generated at the remote device 520 to another device, or to an internet gateway. The remote device 520 may also control irrigation of the areas around the moisture sensors.

At least a portion of the multiple moisture sensors 522, 524, 526 are located under the ground surface 510. The multiple moisture sensors 522, 524, 526 are configured to measure the moisture level in the vicinity of tree 530. It should be noted that the trees are exemplary only and the multiple moisture sensors may be located in multiple liquid absorbing volumes (LAVs) or in different locations in a single LAV. In some exemplary cases, the multiple moisture sensors 522, 524, 526 transmit the electrical signals generated by the transducers using communication cables 523, 525, 527, respectively. In some other cases, the multiple moisture sensors 522, 524, 526 may send the electrical signals using low-range wireless transmitter, for example a blue-tooth transmitter. In some exemplary cases, the multiple moisture sensors 522, 524, 526 may be positioned in various depths under the ground surface 510. For example, multiple moisture sensors may be installed on a single rod inserted into the ground, one sensor disposed 0.7 meters below ground surface 510 and the other sensor disposed 1.7 meters below ground surface 510. The signals provided by the two sensors installed in various depths may assist the person in charge of irrigating the plants to optimize the irrigation plan.

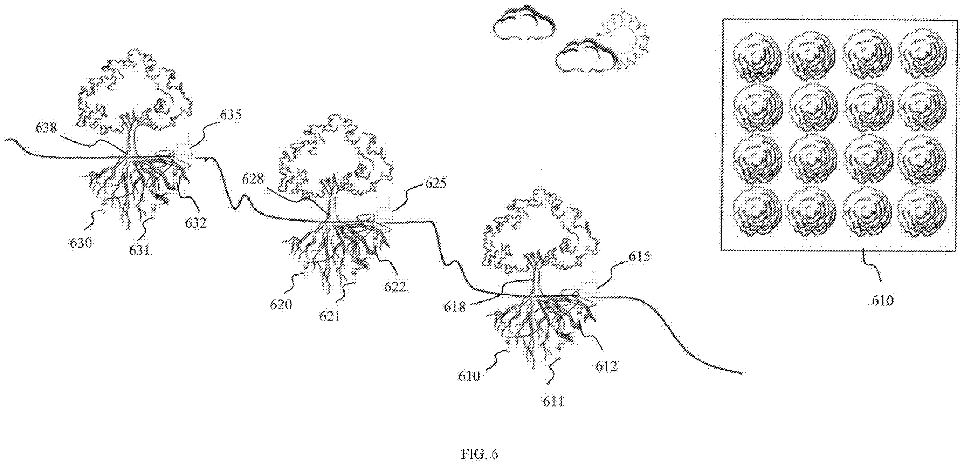

Figure 6 shows multiple moisture sensors located in multiple locations in a specific area, according to exemplary embodiments of the disclosed subject matter. Figure 6 shows multiple trees 618, 628, 638. Each of the trees 618, 628, 638 is associated with one or multiple moisture sensors located in the vicinity of the tree. Tree 618 is associated with moisture sensors 610, 611, 612, tree 628 is associated with moisture sensors 620, 621, 622, and tree 638 is associated with moisture sensors 630, 631, 632. The multiple moisture sensors, for example 610, 611, 612, may be in the same depth or in different depths. The multiple moisture sensors of each tree may transmit the electrical signals to a remote device associated with a single tree. For example, moisture sensors 610, 611, 612 transmit the signals to remote device 615, moisture sensors 620, 621, 622 transmit the signals to remote device 625, and moisture sensors 630, 631, 632 transmit the signals to remote device 635. In some other exemplary cases, a single remote device is associated with an entire area, for example associated with 50 trees. Thus, when each tree is associated with 3 sensors, the remote device may receive signals from 150 sensors.

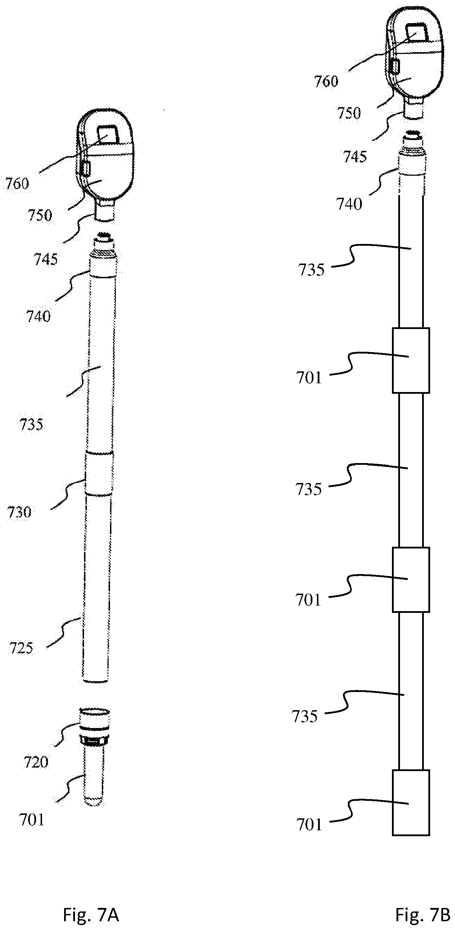

Figure 7A shows a size-adjustable moisture sensor, according to exemplary embodiments of the disclosed subject matter. The moisture sensor's height may be adjusted to enable a user of the sensor to place the liquid container 701 in various depths. For example, the same moisture sensor may be located 20 centimeters below a surface, 50 centimeters below a surface and 80 centimeters below a surface. The liquid container 701 is coupled to a container cover 720 secured to a first housing 725. The first housing may comprise the piston (not shown) and the transducer. The first housing 725 is coupled to a second housing 735 using a first connector 730. The first connector 730 may be a double-sided screw, enabling connecting the first housing 725 from below and connecting the second housing 735 from above. In some embodiments, the first connector is replaced with a moisture sensor apparatus of the invention. In this way there may be multiple sensors in one size-adjustable apparatus. The sensor-housing- sensor pattern may be repeated multiple times as depicted in Figures 7B. Having multiple sensors in one apparatus allows for simultaneous measuring at different depths. Thus, one apparatus can determine irrigation needs at multiple depths in the same location. The second housing 735 is coupled to a transmitter housing extension 745 via second connector 740, which functions similarly to the first connector 730. In some embodiments, multiple sensor-housing repeats are incorporated into the apparatus before transmitter housing extension 745. Optionally there may be a final connector 740 before transmitter housing extension 745, regardless of the number of sensors incorporated into the apparatus. In some embodiments, the apparatus comprises at least one sensor. In some embodiments, the apparatus comprises a plurality of sensors. In some embodiments, the apparatus comprises 1, 2, 3, 4, 5, 6, 7, 8, 9 or 10 sensors. Each possibility represents a separate embodiment of the invention. In some embodiments, the more than one sensors are separated by connectors so that the sensors, when inserted into an LAV are at different depths. In some embodiments, each sensor is at least 0.1, 0.2, 0.25, 0.3, 0.4, 0.5, 0.6, 0.7, 0.75, 0.8, 0.9, 1, 2, 3, 4 or 5 meters apart. Each possibility represents a separate embodiment of the invention. Though it is Figure 7B that depicts an embodiment with multiple sensors in one apparatus, this feature may be applied to any other embodiment of the invention described herein. The transmitter housing extension 745 is coupled to a transmitter housing 750 that houses the wireless transmitter (not shown). In some cases, the transmitter housing 750 is connected to a solar panel 760 installed on the external surface of the transmitter housing 750. The solar panel 760 may be electronically coupled to the wireless transmitter, providing electrical power to the transmitter. In some embodiments, the solar panel is configured to power the apparatus, the sensor, the receiver, the transmitter, or a combination thereof. In some embodiments, the solar panel is located in a region of the apparatus configured to be above ground during operation of the apparatus.

In some exemplary cases, the transmitter housing extension 745 is coupled to the first housing 725 and the second housing 735 is removed. This way, the person using the moisture sensor can adjust the distance between the liquid container 701 and the transmitter and hence the depth of the liquid container in the volume surrounding the moisture sensor. In some other embodiments, the moisture sensor comprises a single housing connecting the transducer and the transmitter housing 750, the single housing is designed in an adjusted manner, for example as a telescopic pole.

By a first aspect there is provided a water potential effector apparatus comprising a water permeable housing and a volume changing material (VCM). The invention provides a water potential effector apparatus comprising a water permeable housing. The water permeable housing [101, 201, 301, 401, 701, 801, 901, 1001, 1101] is configured to be inserted into water containing media, or LAV. In one embodiment, the water containing media is soil. In some embodiments, the housing is water permeable at at least one portion of the housing. In some embodiments, the entire housing is water permeable. In some embodiments, particular regions of the housing are water permeable. In some embodiments, the bottom of the housing is water permeable. In some embodiments, at least one portion of the housing is water permeable. In one embodiment the housing is made of porous media. In some embodiments, the water permeable portion or region is made of porous media. In further embodiment, the porous media is a natural, synthetic or a semi- synthetic membrane. In yet another embodiment, the porous media is clay.

In the embodiments of the invention the housing comprises a Volume Changing Material (VCM) capable of retaining water. In some embodiments, the VCM can absorb water. VCM [100, 200, 300, 400, 800, 900, 1000, 1100] is configured to increase or decrease its volume in response to changes in water potential in the water containing media. In some embodiments, the housing surrounds an area comprising the VCM. In some embodiments, the apparatus of the invention comprises the VCM. In some embodiments, the VCM increases or decreases its volume in response to increases and decreases in water entering the VCM, respectively. In some embodiments, the VCM increases or decreases its volume in response to increasing and decreasing water concentration, respectively. In some embodiments, the water concentration is the concentration of water in the VCM. In some embodiments, the water concentration is the concentration of water is the water containing media. In some embodiments, the water concentration is the concentration of water in the VCM, the water containing media or both. In one embodiment, when water potential in the water containing media is increased, the water passes through the housing to the VCM, causing the VCM to increase in volume. In some embodiments, the increase in VCM volume builds the turgor pressure. In one embodiment, when water potential in the water containing media decreases, the water exits from the VCM through the housing into the water containing media, causing the VCM to decrease in volume. In an embodiment of the invention, the VCM is in direct contact with an inner surface of the housing. In some embodiments, the VCM is in direct contact with the water permeable portion of the housing. In one embodiment, an inner surface of the housing is coated with the VCM. In some embodiments, the entire inner surface of the housing is in contact with or coated with the VCM. In yet another embodiment, only a portion of the inner surface of the housing is in a direct contact with the VCM. In some embodiments, only a portion of the inner surface of the housing is coated with the VCM.

In one embodiment of the invention, when the volume of the VCM increases, it becomes operationally engaged with a receiver [803, 903, 1003, 1103]. In some embodiments, the receiver is a transducer [140, 240, 340]. In some embodiments, the receiver or transducer is configured to translate the force applied by the VCM into an electrical or mechanical signal. In some embodiments, the receiver comprises a diaphragm or a membrane. In some embodiments, the VCM contacts the diaphragm or membrane. The receiver of the invention is, without limitation, pressure sensor, force sensor, valve, and a gauge. In some embodiments, the receiver comprises a sensor. In some embodiments, the sensor is configured to sense the volume of the VCM. In some embodiments, the sensor comprises at least one of a pressure sensors, a force sensor, a valve and a gauge. In some embodiments, the receiver is configured to engage the VCM. In some embodiments, the receiver is configured to engage the VCM at or above a predetermined threshold of water concentration. In some embodiments, the receiver is configured to engage the VCM when it reaches or is greater than a predetermined volume. In some embodiments, the receiver is configured to transduce a signal. In some embodiments, the receiver is configured to transduce a signal when engaged. In some embodiments, the receiver is configured to transduce a signal when disengaged. In some embodiments, the receiver is configured to transduce a signal when engaged and/or disengaged. In some embodiments, the apparatus of the invention further comprises a receiver as described herein.

The housing further comprises a compressible insert [105, 905, 1005, 1105] configured to maintain a predetermined inner pressure. In some embodiments, the housing surrounds an area comprising the insert. The apparatus of the invention comprises the insert. The insert is configured to change its volume in response to the force applied by the VCM. In some embodiments, the insert is configured to change its volume in response to a force applied to it. In some embodiments, the insert is configured to maintain a predetermined pressure within the housing. In some embodiments, the insert is configured to maintain at least a predetermined pressure within the housing. In some embodiments, the insert is configured not to break under the maximum pressure applied by the VCM. The maximum pressure the VCM can exert would occur when it is 100% saturated with water. The VCM applies its lowest pressure when it comprises no water. In such conditions the insert takes up its maximum volume within the apparatus and keeps a minimum predetermined pressure within the apparatus. Maintaining such a minimum pressure is advantageous in that it strengthens the apparatus against potential breakage or deformation. As soil around the apparatus moves or is compressed it can apply a physical force on the housing of the apparatus. As the housing must be water permeable, it is over made of a rigid, or not very strong material, such as clay. If a minimum pressure is not maintained in the apparatus, it can more easily deform under pressure from the surrounding soil, especially if this force is sufficient to overcome the strength of the housing.

In some embodiments of the invention, when the volume of the VCM increases, it pushes on the insert and the insert becomes operationally engaged with a receiver [803, 903, 1003, 1103]. In some embodiments, the receiver or transducer is configured to translate the force applied by the insert into an electrical or mechanical signal. In some embodiments, the insert contacts the diaphragm or membrane. In some embodiments, the insert and/or VCM becomes operationally engaged with a receiver [803, 903, 1003, 1103]. In some embodiments, the receiver or transducer is configured to translate the force applied by the insert and/or VCM into an electrical or mechanical signal. In some embodiments, the insert and/or VCM contacts the diaphragm or membrane.

The insert of the invention may contain gas, liquid, solid, semi-solid material or a combination thereof. In one embodiment of the invention the insert is not permeable to gaseous, solid, semi-solid or liquid media. In some embodiments, the insert comprises a gas-filled volume. In some embodiments, the insert is a balloon or bladder filled with a gas, liquid, solid, semi-solid material or a combination thereof. Each possibility represents a separate embodiment of the invention. In some embodiments, the insert applies a pressure on the VCM that is not sufficient to pushout water from the VCM. In some embodiments, the insert applies a pressure on the VCM that is not sufficient to hinder water's ability to enter the VCM. In some embodiments, at high pressure from the VCM the insert applies a pressure on the VCM that may slow entry of water. In some embodiments, this counter pressure of the insert is considered in calibrating the apparatus.

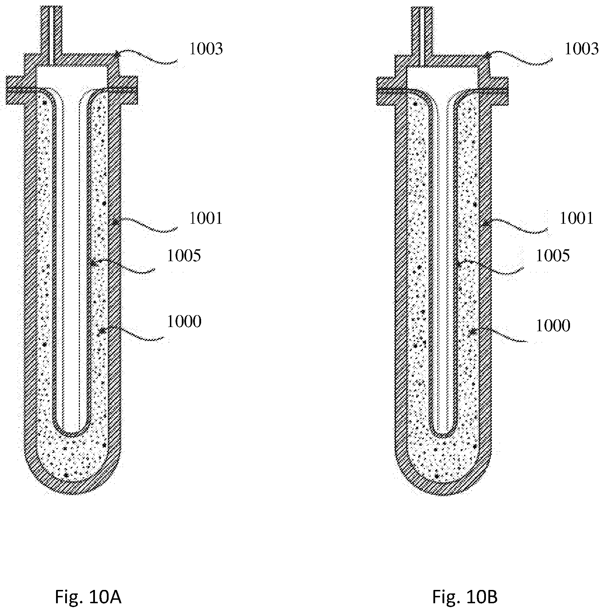

Figures 10A and 10B show another embodiment where the opening of the compressible insert is operatively engaged with the receiver [1003], through direct physical contact. When water permeates into the housing [1001] the volume of the VCM [1000] increases, thus building the turgor pressure inside the housing. The pressure triggers compression of the insert [1005] (as shown in Fig. 10B), which, in turn, triggers an increase in the force applied to the receiver [1003]. The configuration of the insert [105, 1005, 1105] illustrated in Figures IB, 10A-B and 11A-B, is particularly advantageous since it enhances sensitivity of the water potential effector apparatus of the invention and enables sensing of subtle changes in the water potential.

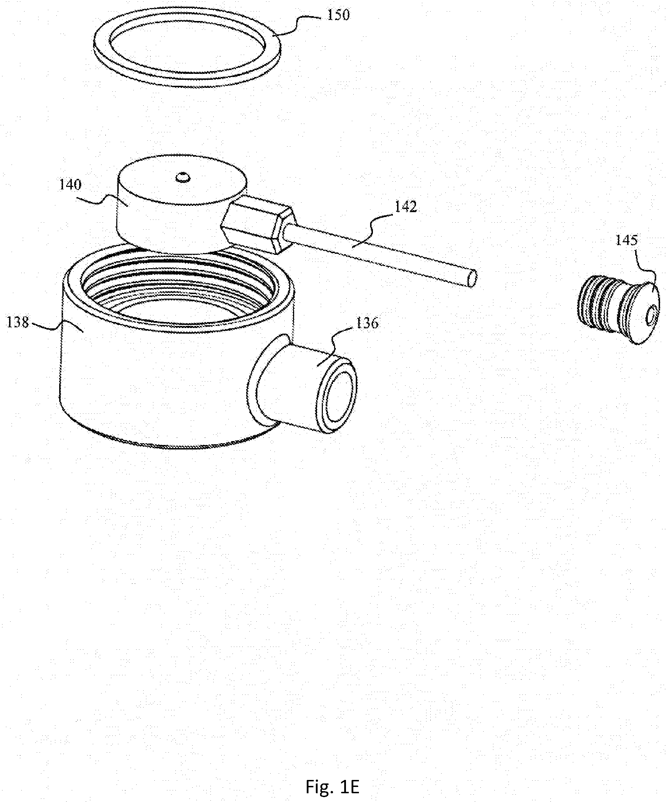

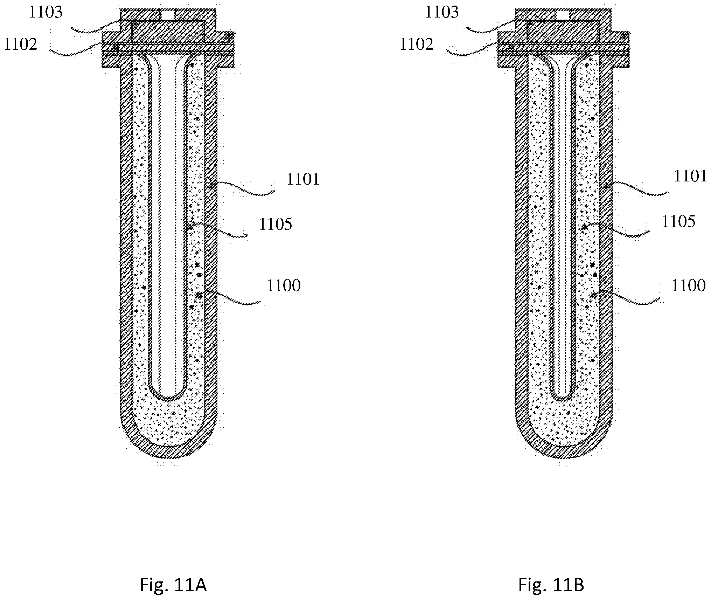

Figures 11 A and 1 IB show embodiments where the opening of the compressible insert is operatively engaged with a receiver through a diaphragm [1102]. The term "opening" refers to the area of contact When water permeates into the housing [1101] the volume of the VCM [1100] increases, thus building the turgor pressure inside the housing. The increasing pressure triggers compression of the insert [1105] (as seen in Fig. 11B), triggering an increase in the force applied to the diaphragm [1102] and, subsequently to the receiver [1103]. In some embodiments, the diaphragm is deformed in response to the force applied. In some embodiments, there is no diaphragm and the force is applied directly to the receiver. In some embodiments, the diaphragm's deformation closes a circuit.

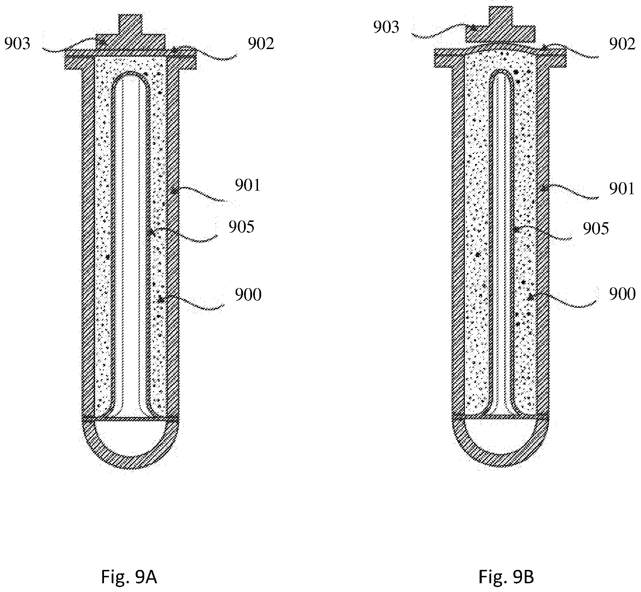

Figures 9A and 9B show yet another embodiment where the opening of the compressible insert [905] is oriented toward the bottom part of the housing [901]. When water permeates into the housing [901] the volume of the VCM [900] increases, thus building the turgor pressure inside the housing. The pressure triggers compression of the insert [905] (as seen in Fig. 9B), and an increase in the force applied to the diaphragm [902] and to the receiver [903]. In some embodiments, the force is applied directly to the receiver and there is no diaphragm.

In some embodiments, the VCM engages the receiver. In some embodiments, the insert engages the receiver. In some embodiments, the VCM and insert engage the receiver. The receiver is configured to engage the VCM, insert, or both. In some embodiments, the receiver engages the VCM, insert or both at a predetermined threshold of water concentration. In some embodiments, the receiver engages the VCM, insert or both at a predetermined threshold of water potential. In some embodiments, only a defined area of the VCM or insert contact the receiver or the diaphragm. In some embodiments, the insert contacts the receiver or diaphragm and the area of contact is smaller than the area of contact of the insert and VCM. A skilled artisan will appreciate that by having a large area of contact between the VCM and the insert a small change in water concentration in the VCM will have an exponentially larger impact on the force exerted on the insert. If a uniform increase in VCM volume occurs around the full surface area of the insert even a small increase will greatly affect the pressure inside the insert. If the area of contact between the insert and the receiver is comparatively small than all this force will be applied at this small area. In this was a very small change in water concentration can result in a large force applied by the insert to the receiver. In some embodiments, the ratio of the area of contact between the insert and VCM to the area of contact between the insert and the receiver is at least 2: 1, 3:1, 4: 1, 5: 1, 6: 1, 7: 1, 8: 1, 9: 1, 10: 1, 15: 1, 20: 1, 25: 1, 30: 1, 35: 1, 40: 1, 45: 1, 50: 1 or 100: 1. Each possibility represents a separate embodiment of the invention. In some embodiments, only a portion of the insert contacts the receiver. In some embodiments, the apparatus comprises a rigid separator that separates a portion of the insert, VCM or both from the receiver, such that only a portion of the insert, VCM or both engages the receiver.

In some embodiments, the insert has a uniform thickness. In some embodiments, the insert has a uniform elasticity. In some embodiments, the insert compresses or inflates at the same rate uniformly across its surface in response to a given pressure. In some embodiments, the insert is not uniform. In some embodiments, at least one part of the insert deforms more easily in response to internal pressure. In some embodiments, at least one part of the insert deforms less easily in response to internal pressure. In some embodiments, the region of the insert in contact with the receiver deforms more easily in response to pressure. In some embodiments, the region of the insert in contact with the receiver deforms less easily in response to pressure.

In some embodiments, the insert is surrounded by the VCM. In some embodiments, the entire length of the insert is surrounded by the VCM. In some embodiments, the insert is surrounded on three sides by the VCM and the fourth side contacts the receiver or diaphragm. In some embodiments, the insert is surrounded on three sides by the VCM and the fourth side contacts the housing. In some embodiments, the internal pressure of the insert and the pressure of the VCM with no water is calibrated such that a predetermined increase in water concentration in the VCM will result in an expansion of the VCM, insert or both necessary to engage the receiver.

In some examples not forming part of the present invention, the housing does not comprise a compressible insert. When water permeates the housing [101], the volume of the VCM [100] increases, thus building the turgor pressure inside the housing. When the VCM is fully swollen, or reaches a predetermined threshold, the force is applied to the diaphragm [102], which in turn, applies the force to the receiver [103]. Alternatively, there is no diaphragm and the force is applied directly to the receiver.

Between 5-35% of the space inside the housing is VCM. In some embodiments, the remained of the volume is taken up by insert. In some embodiments, the inside space is substantially free of empty space.

The VCM is a hydrogel. In some embodiments, the VCM comprises a biocide. In some embodiments, the biocide is a pesticide. In some embodiments, the biocide is an antibiotic. In some embodiments, the biocide kills at least one of mold, bacteria, spores and fungi. In some embodiments, the hydrogel is the biocide.

In examples not forming part of the present invention, the hydrogel is made of homopolymers or copolymers of acrylic acid, methacrylic acid, 2-Bromoacrylic acid, 2-(Bromomethyl) acrylic acid, 2-Ethylacrylic acid, Methacrylic acid, 2-Propylacrylic acid, Sodium acrylate, Sodium methacrylate and its derivatives, or sodium hydroxide or similar hydroxide blended with the monomers. The initial monomer concentration of 5-70% in a water solution, is polymerized with a crosslinker of di-acrylate, or di-acrylamide, or, in examples not forming part of the present invention, with di-vinyl. In one embodiment, the crosslinker concentration is within the range of 0% wt/wt to 20% wt/wt. In examples not forming part of the present invention, the formed polymer is a polymer of polyacrylic acid. In another example not forming part of the present invention, the formed polymer is a polymer of sodium salt of polyacrylic acid.

In further embodiment of the invention, the hydrogel is made of homopolymers or copolymers of Alkylacrylamide, N-(3-Aminopropyl)methacrylamide hydrochloride, N-tert-Butylacrylamide, Diacetone acrylamide, N,N-Diethylacrylamide, N,N- Diethylmethacrylamide, N,N-Dimethylacrylamide, N-[3-(Dimethylamino)propyl]methacrylamide, N-Ethylacrylamide, N,N'-Hexamethylenebis (methacrylamide), N-Hydroxyethylacrylamide, N-(Hydroxymethyl) acrylamide, (4- Hydroxyphenyl)methacrylamide, 2-Hydroxypropyl-methacrylamide, N-(Isobutoxymethyl) acrylamide, N-Isopropylacrylamide, N-Isopropylacrylamide, N-Isopropylmethacrylamide, Methacrylamide and its derivatives.

In one embodiment, copolymers of acrylic acid, acrylamide, maleic anhydride copolymer, polyvinyl alcohol copolymers, cross-linked polyethylene oxide, crosslinked carboxymethylcellulose and starch grafted copolymer are used to control the hydrogel mechanical properties, and its swelling deswelling inside the limiting housing.

In yet another embodiment, the salt derivatives of acrylic acid are used for dry start. In another embodiment the hydrogel further comprises fertilizer salt, pre-dissolved within the hydrogel. Addition of fertilizer increases the osmotic pressure and triggering an increase of at least 80% in swelling from dry state. Fast response of acrylamide derivatives is apparent in salt-rich soil due to the increased osmotic pressure.

In one embodiment, the hydrogel is configured to maintain its thermal stability. In one embodiment conjugated water molecule is present in the polymer. Conjugated water in the polymer perform as antifreeze, lowering the freezing temperature of the gel to -5°C and keeping the hydrogel un-brittle. This temperature is relevant to any soil containing viable plants.

In some embodiments, the apparatus is operatively linked to an irrigation system. In some embodiments, the receiver signals to the irrigation system. In some embodiments, the irrigation system is activated or shutoff by the apparatus. In some embodiments, the irrigation system activates or shuts off in response to the transduced signal. In some embodiments, the signal shuts off the irrigation system. In some embodiments, lack of a signal activates the irrigation system. In some embodiments, when the receiver is not engaged it signals to activate the irrigation system, and when the receiver is engaged it signals to shutoff the irrigation system. In some embodiments, a device is disclosed comprising a soil water potential measuring system and the apparatus. In some embodiments, the apparatus is linked to a memory or storage device. In some embodiments, the apparatus is linked to an above ground readout or display.

In examples not forming part of the present invention, the apparatus is for use in moisture sensing. According to the invention, the apparatus is for use in measuring water potential. In some embodiments, the apparatus is for use in effecting water potential in water-containing media. In some embodiments, the apparatus is inserted in the water-containing media. In some embodiments, the water-containing media is proximal to the apparatus. In some embodiments, the water-containing media is within 1, 2, 3, 4, 5, 7, 10, 15, 20, 25, 30, 35, 40, 45 or 50 meters of the apparatus. Each possibility represents a separate embodiment of the invention.

In the context of the invention, water potential effector apparatus illustrated on Figures 2A-B, 3A-B, and 4A-B; has certain advantages attributed to the compressible insert [201,301,401]. The advantages are, without limitation, improved durability; greater sensing capacity; resistance to extreme temperature conditions, such as frozen soil. For example, the compressible insert allows to control the pressure inside the housing and to withstand sharp increase in VCM volume due to freezing of the soil, thus preventing explosion of the housing. The apparatus of the invention can therefore be inserted into the soil throughout the year and retain its functionality under various climate conditions without any maintenance requirements. An additional advantage attributed to the compressible insert is shortening the time required for the VCM to reach the transducer, and to therefore start generating the desirable output.

The terminology used herein is for the purpose of describing particular embodiments only and is not intended to be limiting of the invention. As used herein, the singular forms "a," "an" and "the" are intended to include plural forms as well, unless the context clearly indicates otherwise. It will be further understood that the terms "comprises" or "comprising," when used in this specification, specify the presence of stated features, integers, steps, operations, elements components and/or groups or combinations thereof, but do not preclude the presence or addition of one or more other features, integers, steps, operations, elements, components and/or groups or combinations thereof. As used herein the terms "comprises", "comprising", "includes", "including", "having" and their conjugates mean "including but not limited to". The term "consisting of means "including and limited to".

As used herein, the term "and/or" includes any and all possible combinations or one or more of the associated listed items, as well as the lack of combinations when interpreted in the alternative ("or").

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and claims and should not be interpreted in an idealized or overly formal sense unless expressly so defined herein. Well-known functions or constructions may not be described in detail for brevity and/or clarity.

It will be understood that when an element is referred to as being "on," "attached" to, "operatively coupled" to, "operatively engaged" with, "connected" to, "coupled" with, "contacting," etc., another element, it can be directly on, attached to, connected to, operatively coupled to, operatively engaged with, coupled with and/or contacting the other element or intervening elements can also be present. In contrast, when an element is referred to as being "directly contacting" another element, there are no intervening elements present.

It will be understood that, although the terms first, second, etc., may be used herein to describe various elements, components, regions, layers and/or sections, these elements, components, regions, layers and/or sections should not be limited by these terms. Rather, these terms are only used to distinguish one element, component, region, layer and/or section, from another element, component, region, layer and/or section.

Certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

## Claims

1. An apparatus for measuring water potential in the soil, comprising:
a. a housing (101) comprising at least one portion that is water permeable, wherein said water permeable portion comprises a porous material;
b. a volume changing material VCM (100) capable of retaining water, wherein said VCM increases or decreases its volume in response to increased or decreased water concentration respectively, and wherein said VCM is in direct contact with an inner surface of said at least one water permeable portion of said housing, and wherein the VCM is a hydrogel having the concentration of 5-35%, wherein the hydrogel is made of homopolymers or copolymers of acrylamide, wherein the initial monomer concentration of 5-70% in a water solution, is polymerized with a crosslinker of di-acrylate, or di-acrylamide, and wherein the hydrogel fills between 10-90% of the volume of space inside said housing;
c. a compressible insert (105) configured to maintain at least a predetermined inner pressure within said apparatus; and
d. a receiver (140) configured to engage the VCM (100), compressible insert (105) or both at or above a predetermined threshold of water concentration and configured to transduce a signal when engaged, when disengaged, or both; and wherein said receiver (140) is configured to translate a force applied by said VCM (100), compressible insert (105) or both into an electrical signal.

2. The apparatus of claim 1, wherein said receiver (140) comprises a pressure sensor, a traveling sensor, a force sensor, a valve, or a gauge.

3. The apparatus of anyone of claims 1 and 2, wherein said porous material is a natural, synthetic, or a semisynthetic membrane.

4. The apparatus of anyone of claims 1 to 3, wherein the compressible insert (105) comprises a gas-filled inner volume.

5. The apparatus of any one of claims 1 to 4, wherein the VCM (100) comprises a biocide.

6. The apparatus of any one of claims 1 to 5, further comprising a diaphragm (125) between said VCM (100), compressible insert (105) or both and said receiver (140), wherein said diaphragm (125) transfers force from said VCM (100), compressible insert (105) or both to said receiver (140).

7. Device comprising a soil water potential system and an apparatus according to any one of claims 1 to 6.

## Patentansprüche

1. Eine Vorrichtung zur Messung des Wasserpotentials im Boden, die folgendes umfasst:
a. ein Gehäuse (101) mit mindestens einem wasserdurchlässigen Teil, wobei der wasserdurchlässige Teil ein poröses Material umfasst;
b. ein volumenveränderndes Material VCM (100), das Wasser zurückhalten kann, wobei das VCM sein Volumen in Reaktion auf eine erhöhte bzw. verringerte Wasserkonzentration vergrößert oder verkleinert und wobei das VCM in direktem Kontakt mit einer Innenfläche des mindestens einen wasserdurchlässigen Abschnitts des Gehäuses steht, und wobei das VCM ein Hydrogel mit einer Konzentration von 5-35% ist, wobei das Hydrogel aus Homopolymeren oder Copolymeren von Acrylamid hergestellt ist, wobei die anfängliche Monomerkonzentration von 5-70% in einer Wasserlösung mit einem Vernetzer aus Diacrylat oder Diacrylamid polymerisiert ist und wobei das Hydrogel zwischen 10-90% des Raumvolumens innerhalb des Gehäuses ausfüllt;
c. einen komprimierbaren Einsatz (105), der so gestaltet ist, dass er mindestens einen vorbestimmten Innendruck in der Vorrichtung aufrechterhält; und
d. einen Empfänger (140), der so gestaltet ist, dass er mit dem VCM (100), dem komprimierbaren Einsatz (105) oder beiden bei oder oberhalb eines vorbestimmten Schwellenwerts der Wasserkonzentration in Eingriff kommt und so gestaltet ist, dass er ein Signal überträgt, wenn er in Eingriff kommt, wenn er außer Eingriff kommt oder beides; und wobei der Empfänger (140) so gestaltet ist, dass er eine von dem VCM (100), dem komprimierbaren Einsatz (105) oder beiden ausgeübte Kraft in ein elektrisches Signal umsetzt.

2. Die Vorrichtung nach Anspruch 1, wobei der Empfänger (140) einen Drucksensor, einen Wegsensor, einen Kraftsensor, ein Ventil oder ein Messgerät umfasst.

3. Die Vorrichtung nach einem der Ansprüche 1 und 2, wobei das poröse Material eine natürliche, synthetische oder halbsynthetische Membran ist.

4. Die Vorrichtung nach einem der Ansprüche 1 bis 3, wobei der komprimierbare Einsatz (105) ein gasgefülltes Innenvolumen aufweist.

5. Die Vorrichtung nach einem der Ansprüche 1 bis 4, wobei das VCM (100) ein Biozid enthält.

6. Die Vorrichtung nach einem der Ansprüche 1 bis 5, die ferner eine Membran (125) zwischen dem VCM (100), dem komprimierbaren Einsatz (105) oder beiden und dem Empfänger (140) umfasst, wobei die Membran (125) eine Kraft von dem VCM (100), dem komprimierbaren Einsatz (105) oder beiden auf den Empfänger (140) überträgt.

7. Gerät mit einem System zur Messung des Wasserpotentials im Boden und einer Vorrichtung nach einem der Ansprüche 1 bis 6.

## Revendications

1. Un appareil pour mesurer le potentiel hydrique du sol, comprenant :
a. un boîtier (101) comprenant au moins une partie perméable à l'eau, dans lequel ladite partie perméable à l'eau comprend un matériau poreux ;
b. un matériau à volume variable VCM (100) capable de retenir l'eau, dans lequel ledit VCM augmente ou diminue son volume en réponse à une augmentation ou une diminution de la concentration d'eau respectivement, et dans lequel ledit VCM est en contact direct avec une surface intérieure de ladite au moins une partie perméable à l'eau dudit boîtier, et dans lequel le VCM est un hydrogel dont la concentration est comprise entre 5 et 35 %, dans lequel l'hydrogel est constitué d'homopolymères ou de copolymères d'acrylamide, dans lequel la concentration initiale de monomère de 5 à 70 % dans une solution d'eau est polymérisée avec un réticulant de di-acrylate ou de di-acrylamide, et dans lequel l'hydrogel remplit entre 10 et 90 % du volume de l'espace à l'intérieur dudit boîtier ;
c. une pièce rapportée compressible (105) configurée pour maintenir au moins une pression intérieure prédéterminée à l'intérieur de l'appareil ; et
d. un récepteur (140) configuré pour engager le VCM (100), la pièce rapportée compressible (105) ou les deux à ou au-dessus d'un seuil prédéterminé de concentration d'eau et configuré pour transmettre un signal lorsqu'il est engagé, lorsqu'il est désengagé, ou les deux ; et dans lequel ledit récepteur (140) est configuré pour traduire une force appliquée par ledit VCM (100), la pièce rapportée compressible (105) ou les deux en un signal électrique.

2. L'appareil selon la revendication 1, dans lequel ledit récepteur (140) comprend un capteur de pression, un capteur de déplacement, un capteur de force, une soupape ou une jauge.

3. L'appareil selon l'une quelconque des revendications 1 et 2, dans lequel ledit matériau poreux est une membrane naturelle, synthétique ou semi-synthétique.

4. L'appareil selon l'une quelconque des revendications 1 à 3, dans lequel la pièce rapportée compressible (105) comprend un volume intérieur rempli de gaz.

5. L'appareil selon l'une quelconque des revendications 1 à 4, dans lequel le VCM (100) comprend un biocide.

6. L'appareil selon l'une quelconque des revendications 1 à 5, comprenant en outre un diaphragme (125) entre ledit VCM (100), la pièce rapportée compressible (105) ou les deux et ledit récepteur (140), dans lequel ledit diaphragme (125) transfère la force dudit VCM (100), de la pièce rapportée compressible (105), ou des deux, vers ledit récepteur (140).

7. Dispositif comprenant un système de mesure du potentiel hydrique du sol et un appareil selon l'une quelconque des revendications 1 à 6.
